# NEW EUROPEAN PATENT SPECIFICATION

(11) **EP 0 707 841 B2**
(45) Date of publication and mention of the opposition decision: **10.05.2006**
(45) Mention of the grant of the patent: 14.04.1999
(21) Application number: 94203040.4
(22) Date of filing: 20.10.1994
(51) Int. Cl.: A61F 13/15

(54) **Process to provide material connections for absorbent articles by soldering**
Verfahren zum Verbinden von absorbierenden Artikeln durch Löten
Procédé pour assembler des articles absorbant par soudage

(43) Date of publication of application: 24.04.1996
(73) Proprietor: THE PROCTER & GAMBLE COMPANY, Cincinnati, Ohio 45202 (US)
(72) Inventor: Geilich, Ralf, D-74564 Crailsheim-Rossfeld (DE); Plumley, Julian Ashton, D-74589 Satteldorf (DE)
(74) Representative: Kremer, Véronique Marie Joséphine

(56) References cited:
- EP-A- 0 196 654
- EP-A- 0 293 065
- WO-A-93/09744
- WO-A-93/11725
- US-A- 4 146 586
- US-A- 4 156 398
- US-A- 4 184 902
- US-A- 4 576 997
- US-A- 4 585 819
- US-A- 4 726 976
- US-A- 4 778 458
- US-A- 4 911 948
- US-A- 4 973 326
- US-A- 5 064 492
- DATABASE WPI Derwent Publications Ltd., London, GB; AN 94-347336 & JP-A-6 271 832 (SEKISUI CHEM IND CO LTD) 27 September 1994
- Techn. Merkblatt H.B. Fuller GmbH
- http://www.scs.uiuc.edu/-che390/report2/rep 2prog04.htm
- Prospekt Winkler + Duennebier

## Description

### Field of the invention

The present invention relates to a process for making disposable absorbent articles providing connections of the materials making up disposable absorbent articles. In particular baby diapers, incontinence products and sanitary napkins which are fabricated in continuous processes are beneficially held together by soldering the materials where connections of them are desired.

### Background of the invention

Disposable absorbent articles like baby diapers, incontinence products and sanitary napkins are typically manufactured from a series of materials. During manufacturing the articles have to be joined to each other to form the respective disposable absorbent article. The material connections created thereby have to satisfy certain requirements a key one of which is the strength of the connection, which needs to be sufficiently high to prevent the products from falling apart at least prior to disposal thereof.

Typically adhesives, especially hot melt adhesives have been successfully used for that purpose. A large variety of adhesives for the different application situations occurring in the manufacturing process of disposable absorbent articles has been developed and continue to be developed. Alternative joining methods include welding which is used between plastic materials of similar kinds allowing to create areas were the materials are fused to each other to create permanent connections.

It now has been found that an alternative method of joining materials, namely soldering, can beneficially be used in the field of absorbent articles. This method as such is known from the art of joining metals but so far has not been applied in the manufacturing of disposable absorbent articles. An off-line process to create composit materials which comprise an an adhesive which is non-tacky at temperatures up to 20°C is disclosed in US 5, 064, 493.

A well known problem occurring when adhesively glueing materials in used in disposable absorbent articles to each other is that adhesive can migrate through permeable materials. This may happen while the adhesive is applied and also thereafter. If unpermeable materials are joined to each other the stress and strain they experience can create small apertures through which the adhesive then migrates.

Adhesives used in the field of disposable absorbent articles are tackified to display adhesive characteristics over a broad range of temperatures. They are usually still tacky at room temperature which makes their presence on the outside of a material used in disposable absorbent articles undesirable.

It is undesirable during the manufacturing process to have adhesive on the outside of any of the materials used in disposable articles because adhesive build up, in particular sticky adhesive build up on fast moving machinery parts quickly leads to unstable process conditions. As a minimum the occurrence of these conditions require frequent cleaning or even cause disastrous material destruction leading to machine stops and reduced efficiency.

Also adhesive which may be present on the outside of disposable absorbent articles is highly undesirable for the consumer. If adhesives which are tacky at the usage temperature of these products, that is body temperature of the wearer of such products, contact the wearer's skin or garment they will usually cause residue adhesive on garments or irritation to the wearer's skin.

These drawbacks of adhesives can be overcome by the alternative process of welding. Welding of materials usually requires material similarities if not identity in order to be feasible at all. Welding also has the disadvantage of requiring very exact temperature controls in order to provide a proper bond between materials while not causing melting away of materials or so called welding holes. This problem of very tight controls or material incompatibility to welding altogether is significantly reduced with today's adhesive technology which however carries the above mentioned draw backs.

It is therefore an objective of the present invention to provide a method allowing to join materials which are usually used in the art of making disposable absorbent articles as an alternative to welding and an alternative to adhesive glueing. It is another objective of the present invention to provide an improvement in that the alternative process developed according to the present invention can be carried out at temperatures similar to those of today's adhesive materials, while eliminating the problems assouated with the stickiness of adhesives below their application temperature.

These and other objectives of the present invention will become more pronounced from the further description of the invention below.

### Summary of the invention

The present invention relates to a process to manufacture disposable absorbent articles in accordance with claim 1. The disposable absorbent articles comprise an absorbent structure which has a first and a second outermost surface located on opposite sides of the absorbent structure and the process comprises the steps of
- providing a first and a second material to be used in the disposable absorbent article;
- conveying the first and the second material in a direction defined as the machine direction;
- joining the first and the second material by soldering to provide a permanent connection.

The solder used in the soldering step of the present invention is a solder which is non-sticky at 20°C, preferably it is non-sticky already at 40°C. The solder application temperature typically is above the solidifying temperature of the solder.

In a preferred embodiment of the present invention the process is used to combine materials which are used in sanitary napkins or panty liners having side flaps. The sanitary napkins or panty liners are used in the crotch portion of an undergarment with the side flaps extending beyond the side edge of the crotch portion of the undergarment and are folded around that side edge for additional soiling protection. Such side flaps can be rendered at least in some parts extensible for improved fit around the side edge by high speed deformation processes.

The materials used in general in the disposable absorbent articles according to the present invention are conveyed in the machine direction at a high speed such as at a surface speed of at least 0.4 m/s and preferably of at least 1.5 m/s. This translates into a production quantity per minute of at least 120 products of 20 cm length per product or for the more preferred speed limit of at least 450 products of 20 cm length per product respectively.

The materials to be combined by soldering in the disposable absorbent articles include any combination of the liquid permeable topsheet the backsheet, preferably a liquid impermeable material, the core and its internal individual layers (if any) as well as multiple layers forming the topsheet or the backsheet.

### Brief description of the drawings

Figure 1 is an exploded view of a sanitary napkin made according to the present invention not showing the solder but identifying the various elements of the preferred sanitary napkins or panty liners as representative examples of disposable absorbent artides.
Figure 2 is an alternative sanitary napkin having a layered absorbent structure.
Figure 3 is yet another alternative sanitary napkin having side flaps.

According to the present invention a disposable article for absorbing liquid by placing it adjacent a body discharge area is joined at least partially by soldering. The article has a body-facing surface, typically provided by a liquid permeable substrate of fibrous or film-like structure; a garment facing surface, preferably provided by a liquid impermeable, but breathable substrate and an absorbent structure placed between the body facing surface and the garment facing surface. The absorbent article has a longitudinal and a lateral axis and can comprise any of the components or features usual in the art including in particular side flap components and any sort of extensibility or elastication feature known in the art.

### Detailed description of the invention

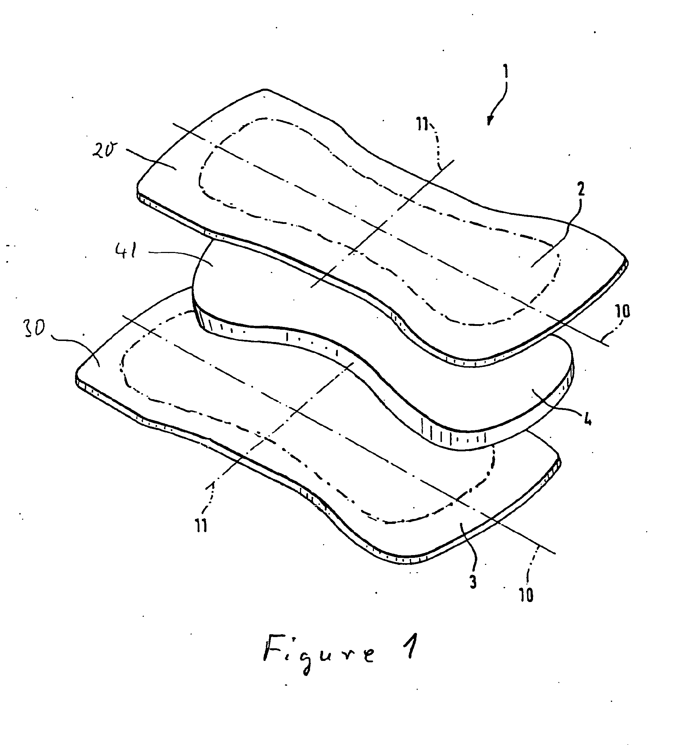

The disposable article for absorbent liquid is described below by reference to a sanitary napkin or panty liner. However products such as adult or baby diapers or adult incontinence inserts can similarly be supplied with the characterising feature of the present invention. Three alternative preferred embodiments of a sanitary napkin or panty liner, made according to the present invention, are shown in an exploded view in Figures 1 to 3. Each of them shows a liquid permeable topsheet (2) overlaying an absorbent structure (4) which overlays a liquid impermeable backsheet (3).

### Topsheet

The topsheet (2) is compliant, soft feeling, an non-irritating to the wearers skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness. A suitable topsheet can be manufactured from a wide range of materials such as woven and non woven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers.

Preferred topsheets for use in the present invention are selected from high loft nonwoven topsheets and apertured formed film topsheets. Apertured formed films are especially preferred for the topsheet because they are pervious to body exudates and yet non absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,394. Particularly preferred microapetured formed film topsheets are disclosed in U.S. patent 4,609,518 and U.S. patent 4,629,643. A preferred topsheet for the present invention comprises the formed film described in one or more of the above patents and marketed on sanitary napkins by The Procter & Gamble Company of Cincinnati, Ohio as "DRI-WEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The body surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT-publication WO 93/09741. Alternatively, the body surface of the topsheet can be made hydrophilic by treating it with a surfactant such as is described in U.S. 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

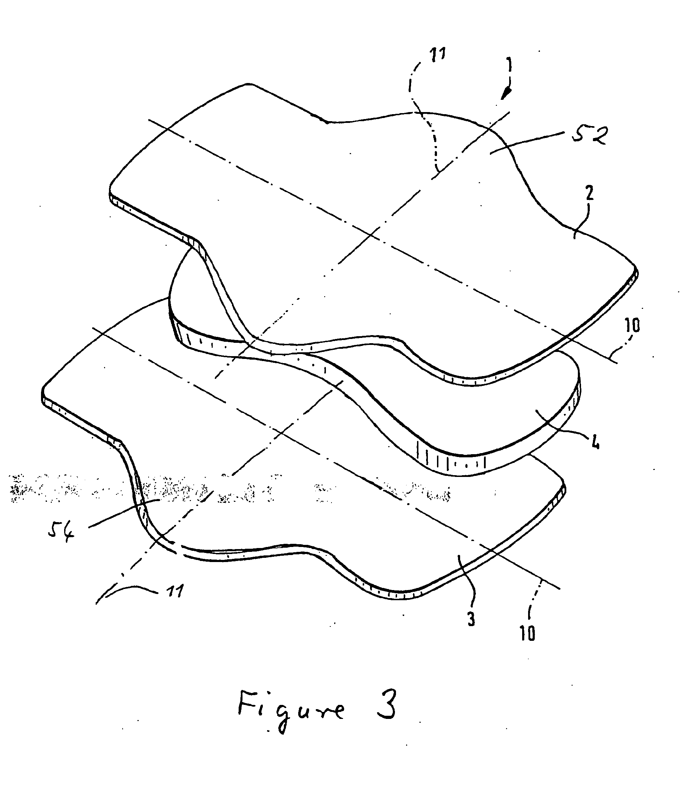

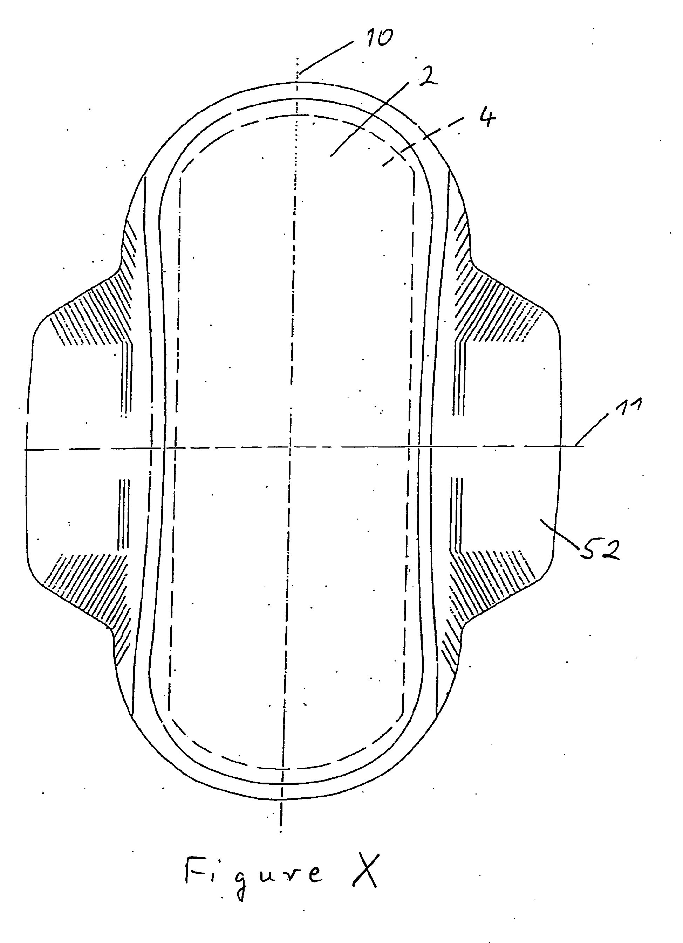

The topsheet (2) typically extends across the whole of the absorbent structure (4) but may extend outside the area coextensive with the absorbent structure (4). As indicated in Figure 1 a peripheral edge portion (20) to be used for joining the topsheet (2) to the back sheet (3) can be provided. Also the topsheet (2) can extend and form part or all of the preferred side flaps as shown and designated 52 in Figure 3.

When referring to the topsheet a multi layer structure or a mono layer structure are contemplated. If the various layers of multilayer topsheets are joined to each other they are as such susceptible to the joining process according to the present invention.

The hybrid topsheet mentioned above is such a multi layer design but other multilayer topsheets such as primary and secondary topsheet designs are also considered susceptible to be joined by the process according to the present invention.

### Absorbent structure

The absorbent structure is shown as a single entity (4) in Figure 1 to 3. It can include the following components: (a) optionally a primary fluid distribution layer preferably together with a secondary fluid distribution layer; (b) a fluid storage layer; (c) optionally a fibrous ("dusting") layer underlying the storage layer; and (d) other optional components. a Primary/Secondary Fluid Distribution Layer

One optional component of the absorbent structure according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent" "hydrocalloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross linked, partially neutralised, polymeric gelling material. This material forms a hydrogel upon contact with water. Such polymer materials can be prepared from polymerizable, unsaturated, acid-containing monomers. Suitable unsaturated acidic monomers for use in preparing the polymeric absorbent gelling material used in this invention include those listed in U.S. Patent 4,654,039 reissued as RE 32,649. Preferred monomers include acrylic acid, methacrylic acid, and 2-acrylamido-2-methyl propane sulfonic acid. Acrylic acid itself is especially preferred for preparation of the polymeric gelling material.

Suitable carriers include materials which are conventionally utilised in absorbent structures such as cellulose fibers, in the form of fluff and /or tissues. Suitable carriers can be used together with the absorbent gelling material, however they can also be used alone or in combinations. Most preferred are tissue or tissue laminates in the context of sanitary napkins /party liners.

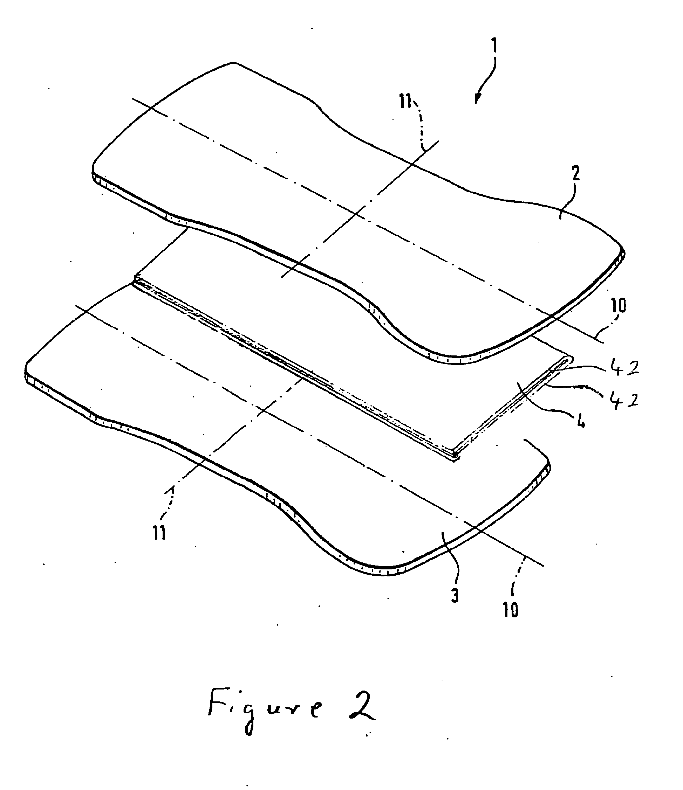

An embodiment made according to the present invention is shown in Figure 2 where the absorbent structure (4) comprises a double layer (42) tissue laminate formed by folding the tissue onto itself. These layers can be joined by soldering both layers to each other.

Modified cellulose fibers such as the stiffened cellulose fibers can also be used. Synthetic fibers can also be used and include those made of cellulose acetate, polyvinyl fluoride, polyvinylidene chloride, acrylics (such as Orion), plyvinyl acetate, non-soluble polyvinyl alcohol plyehtylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., that lower rewet problems.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent structure according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent structure. Indeed, in those instances where the absorbent gelling material is in the form of macro structures such as fibbers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent structure according to the present invention can indude other optional components normally present in absorbent webs. For example, a reinforcing scrim can be positioned within the respective layers, or between the respective layers, of the absorbent structure. Such reinforcing scrims should be of such configuration as to not form interfacial barriers to fluid transfer, especially if positioned between the respective layers of the absorbent structure. Given the structural integrity that usually occurs as a result of thermal bonding, reinforcing scrims are usually not required for the absorbent structures according to the present invention.

Another component which can be included in the absorbent structure according to the invention and preferably is provided close to or as part of the primary or secondary fluid distribution layer are odor control agents. Typically active carbon coated with or in addition to other odor control agents, in particular suitable zeolite or day materials, are optionally incorporated in the absorbent structure. These components can be incorporated in any desired form but often are included as discrete particles.

### Backsheet

The backsheet (3) primarily prevents the exudates absorbed and contained in the absorbent structure from wetting articles that contact the absorbent product such as underpants, pants, pyjamas and undergarments. The backsheet (3) is preferably impervious to liquids (e.g. , menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure but may extend outside the area coextensive with the absorbent structure (4). As indicated in Figure 1 a peripheral edge portion (30), to be used for joining the backsheet (3) to the topsheet (2), can be provided. Also the backsheet can extend into and form part of or all of the preferred sideflaps as shown and designated 54 in Figure 3.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more dothlike appearance. Further, the backsheet can permit vapors to escape from the absorbent structure, i.e. be breathable, while still preventing exudates from passing through the backsheet Also breathable backsheets comprising several layers which are joined to each other can be used and are preferably joined by the method according to the present invention.

### Optional components of the absorbent structure

Optionally, the absorbent product of the present invention can comprise all those components typical for the particularly intended product-use. For example sanitary napkins and panty liners often and preferably comprise components such as sideflaps and panty fastening adhesives in order to improve their positioning and soiling protection performance. Especially side flaps have become very popular with consumers. They can be formed separately and joined to the main portion of the sanitary napkin by the process according to the present invention. Alternatively the side flaps can be formed integrally and are most preferably formed integrally with the topsheet (2) and the backsheet (3) by joining the lateral side flap extensions (52,54) of topsheet (2) and backsheet (3) to each other. The function of side flaps, whether integral or joined to the article after being formed separately, can be further improved by rendering them extensible in one or both directions parallel to the longitudinal axis (10) or lateral axis (11). The extensibility can be provided across all or only part of the side flaps and can be achieved by joining an elastic material to the side flap (for elastication and extensibility) or pleating or ring-rolling those parts which are to be rendered extensible.

If side flaps are formed from laminates - such as those shown in Figure 3 - the soldering according to the present invention can be used particularly beneficial. It eliminates the problems associated with built up of tacky adhesive on high speed equipment parts typically used.

Leg elastication by one or several elastic strands is also common in the art of absorbent products. In general, all typically used components in absorbent products can also be comprised in the absorbent products made according to the process of the present invention.

### Process steps according to the present invention

The process steps of providing at least two materials for being joined in accordance with the present invention requires at least two of the materials or structural entities discussed above for sanitary napkins to be provided. Usually the materials are provided at a fairly high surface speed as roll goods but maintain their speed of converting while forming structural entities to be combined with other materials or other structural entities thereafter.

The surface speed at which these products are made and thereby at which the joining process needs to take place is preferably at least 0.4 m/s and more preferably at least 1.5 m/s. The movement is typically in the longitudinal direction which therefore is parallel to the machine direction of the absorbent articles made according to the present invention.

Movements parallel to the transverse axis (11) are also possible but less often used. The key step according to the present invention is the joining of at least two of the materials provided and conveyed according to the present invention by soldering them to each other. The soldering is considered useful only to provide permanent connections between materials since the formed structures typically have to be coherent to be useful, at least prior to disposal of the disposable absorbent articles.

The soldering must withstand at least a peel strength of 0.4 N/2,5 cm. The peel strength is the strength required to peel apart the materials joined to each other on a sample strip of 2,5 cm width. A full test description is included in the following. The soldering strength can of cause not exceed the material strengths of the materials joined. Therefore an alternative to the test against 0.4 N/2.5 cm can be the internal cohesion test. In this test a connection is delaminated. The soldered connection satisfies the test if the materials are destroyed.

Joining materials by soldering requires to apply a solder to one or both surfaces to be joined and bringing the surfaces into contact before the solder cools below its solidifying temperature. In order to apply the solder it is heated to a temperature above its solidifying temperature and applied in a similar fashion or the same fashion as adhesives are applied. As a matter of fact the same equipment used today for applying adhesives can be adapted to apply the solder to the respective surface where it is needed. Methods like contact coating, roll coating, coating by spraying in random or designed patterns (such as swirl coating,) can all be used to apply the solder according to the present invention.

When bringing the surfaces to be joined together the solder contacts both surfaces intimately at a temperature above its solidifying temperature. It creates a permanent connection after cooling below the solidifying temperature. Incorporated within the word soldering according to the present invention are methods which in the metal art are referred to as braising where the solder forms intermaterial bonds across the contact surface. While not wishing to be limited by theory it is believed that the solder forms a thin layer of intermaterial connections where the top molecular layers of the materials to be soldered to each other are involved.

In order to distinguish the solder from adhesives a stickiness test for defining a solder is described below. Adhesives can be distinguished from the solder according to this test thereby allowing to better understand and repeat the present invention.

Solder materials can be e.g. adhesive materials without the tackifier additives usually used. Alternatively wax type materials of high coherent strength with melting points similar to those of hot melt adhesives can be employed. One such solder example is a cleaning material designated Fuller HS350 New, available from the H.B.Fuller Company, Lueneburg, Germany. The operating temperature of this solder is about 120°C to 140°C while comparable hot melt adhesives such as Findley 990-374-b, available from Findley, Rosendaal, The Netherlands, have operating temperatures of about 135° to 145° C.

### Test methods:

All tests conducted in respect to the present invention require test conditions of 21° plus minus 1° C and relative humidity of 50% unless stated otherwise. All test materials are conditioned at this temperature and humidity for at least 4 hours prior to the test itself.

### Peel strength or peel force testing

The peel strength test analyses the force required to delaminate a connection between materials when one material is peeled from the other material at a 180 degree angle. In respect to defining a permanent connection it has been found most sensible to create a realistic sample of the soldered material connection and analyse the peel strength between the actual materials to be soldered to each other rather than attempt to standardise the materials or solder application. The test is conducted on a sample strip of 2.5 cm width having sufficient end tabs to apply the peel force equally across the whole width of the sample to be tested.

The peel strength of a soldered connection between materials is sufficient if the force required to delaminate the soldered connection is 0.4 N/2.5 cm or if the connection can withstand holding a load of 40g without delaminating.

As already indicated materials having a cohesive strength less than the required 0.4 N/2.5 cm are still considered permanently connected by soldering if the material experiences destructive failure rather than the soldered connection. Obviously this test is substantially easier in executing than the force measurement of the peel strength. It can also be used on materials having higher cohesive strength than 0.4 N/2.5 cm provided one of the materials and not the soldered connection is destroyed. Therefore in general destruction of one of the materials soldered together implies that the soldered connection was permanent

### The stickiness test

The stickiness test distinguishes a solder material from a tacky adhesive. Typically the distinction will be quite apparent when simply touching an open surface of a solder material versus an open surface of an adhesive. However in order to define the distinction the following test methodology has to be applied.

### Principal stickiness test method steps

- melt the test solder material to be analysed for stickiness according to the supplier instruction;
- apply the molten test solder material to a substrate;
- let the molten test solder material and substrate cool to the test temperature while ensuring that the open side of the test material remains untouched and is only exposed to clean air;
- apply a second substrate, the stickiness substrate, to the untouched side of the test material under a certain pressure;
- measure the force required to peel the second substrate from the first substrate.

A solder material will have a peel force of zero at the test temperature while test materials having a peel force greater than zero are not useful in the process according to the present invention.

The test material should be heated/molten to the temperature according to the manufacturers instruction of the test material. Care should be taken with test solders which undergo curing after being heated and cooled down. Even so they may appear sticky prior to heating they can be non-sticky after being applied; i.e. after curing.

The test solder material to be checked is applied to a substrate in the usual way in which adhesives would be applied. For the current test method it has been found that using a Nordson slot coater with a 50mm wide nozzle available from a Nordson, Lueneburg, Germany is useful. The solder is applied for this test in a width of at least 2.5 cm full surface coating at a speed of the first substrate of at least 0.5 m/s relative to the coating equipment. Wider coatings are acceptable while coatings of smaller width or only fractions of the width being coated (e.g. by spiral coating) are non-acceptable in the context of this test. Of course such coating patterns can be used for applying the solder in the particular application context according to the process of the present invention.

The length of the solder material applied should be sufficiently long to conduct a series of tests in order to provide at least ten samples. The individual test sample length should be no less than 10 cm. The amount of solder used according to the stickiness test method should be the same as intended to be used in the desired process according to the present invention. A minimum quantity is however such that the test surface onto which the solder is applied is fully covered by the test solder, and amounts of 10 to 30 g/m² have been found useful.

The first substrate onto which the solder is applied according to the test should be a polyethylene film having a thickness of between 20 and 30 micrometers. Solders having application temperatures too high to be applied to polyethylene films will usually not be considered at all in the context of absorbent articles. Should however in a specific case a solder be considered having an application temperature so high that the polyethylene film would not be able to withstand the application of the test solder (e.g. bum through) then a polypropylene film, or in extreme cases even a polyethylene-terephthalate film can be substituted. It should however be considered that solders having a too high application temperature will not be useful in the context of disposable absorbent articles and typical application temperatures are well below 200°C, usually below 150°C; except for soldering tissue materials useful in manufacturing disposable absorbent articles.

After the solder has been applied to the substrate it is allowed to cool to the temperature at which measurement are to be taken. It has been found that one critical temperature is about 20°C since most manufacturing processes run around room temperature, i.e. about 20°G. The other typically critical temperature is the body temperature of the wearer of disposable absorbent articles since according to the objectives of the present invention any stickiness of the solder to the skin of the wearer should be prevented including a safety margin 40°C is considered to be an adequate upper limit below which the solder matertal has to be non-sticky according to this stickiness test to achieve this objective. Therefore two qualifies of solder can be distinguisfied one which is non-sticky at 20°C or below and one preferred quality which is non-sticky at 40°C or below.

The next step according to the test method is the application of a second substrate. The second substrate should extend to the full surface of the solder test samples and preferably exceed the test surface sufficiently to provide edges where it can be manipulated.

Ideally the second substrate should provide the surface onto which the solder should display its tack of stickiness. However for test standardisation two materials are considered critical.

One substrate material is a woven cation surface. It can be obtained under the designation "white, 100% cotton weave, style # 429-W, available from Loaffler Sitter Technic GmbH, Nettersheim, Germany. The second alternative substrate material is a polyethylene film of 25 micrometer thickness available under the designation "Tacolin Polyethylene Film Code ST 400" available from Taco Plastics, Manchester, Great Britain.

Ten samples each are tested by applying the second substrate to the open surface of the solder under a pressure of 0.4 N/cm. The application of the pressure can be conducted by using a weight in accordance with the size of the test surface area (for the minimum test surface area this weight would be 1 kg) and extending essentially to the test surface.

Upon application of the test weight care should be taken that no uneven weight distribution is introduced e.g. by filting the weight. For the preferred solders according to the present invention the test result will not after over a wide range of compressions of up to 4 N/cm² or even 10 N/cm².

The compression under which the second substrate is applied to the solder surface should be maintained for at least 30 seconds up to 1 minute. The weight is than removed and the samples are left for another 30 seconds to 1 minute.

The measurement of peel strength then can be conducted by attempting to peel one surface away from the other and measuring the required force with highly sensitive force gauges. This measurement is well known in the art to distinguish between the strength of different edhesives. Due to the attempt to measure a zero force value (i.e. non-stickiness) the force measurement creates substantial problems in respect to the accuracy of the results. When measuring an absolute value of zero the variation, also known as measurement noise, is strongly amplified. Therefore it has been found that the stickiness test identifying the solder is satisfied if the second substrate determinates under its own gravitational force.

This is measured by taking the sample, preferably white touching only the outer edges of the first substrate, and turning the sample such that the second substrate is held under its own gravitational force only by the stickiness of the test solder. If the second substrate determinates and talls off within less than 5 seconds, preferably right away, the test solder satisfies the criterium of ran-stickiness.

Samples where the second substrate remains joined to the first substrate are not considered solder materials in the content of the present invention. For these samples a value of stickiness can be measured as indicated above. Of course great care must be taken to ensure that no other forces (e.g. electrostatic forces) are involved in particular if the test is conducted with polyethylene films as the two substrates.

## Claims

1. Process to manufacture disposable absorbent articles, said articles comprising an absorbent structure, said absorbent structure comprising a first and second outermost surface located on opposite sides of said absorbent structure, said process comprising the steps of
- Providing a first and a second material, and a solder, said solder being non-sticky at 40°C
- Conveying said first and said second material in a machine direction at a surface speed of at least 0.4m/s
- Joining said first and said second material by
- Heating the solder to a temperature above the solidifying temperature of said solder
- Applying said solder to one or both materials before said solder cools below its solidifying temperature
- Bringing said first and said second material into contact before said solder cools below its solidifying temperature to provide a permanent connection, and
- said first material is a liquid permeable topsheet and said second material is a backsheet, preferably a liquid impermeable backsheet, or
- said first material is a liquid permeable topsheet and said second material is said absorbent structure and said topsheet and said absorbent structure are joined across said first outermost surface, or
- said first material is a first layer of a multilayer, liquid permeable topsheet and said second material is a second layer of a multi-layer, liquid permeable topsheet, or
- said first material is a breathable backsheet and said second material is said absorbent structure and said backsheet and said absorbent structure are joined across said second outermost surface, or
- said first material is a first layer of a multilayer backsheet and said second material is a second layer of a multi-layer backsheet, preferably said backsheet is liquid impermeable, or
- said absorbent structure comprises multiple layers and said first material is a first layer of said absorbent structure and said second material is a second layer of said absorbent structure.

2. Process according to claim 1 wherein said materials have a surface speed of at least 1.5m/s in machine direction.

3. Process according to claim 1 wherein said topsheet and said backsheet comprise a peripheral edge portion extending beyond the periphery of said absorbent structure and said soldering joins said backsheet to said topsheet in at least part of said peripheral edge portion, preferably along the whole peripheral edge portion.

4. Process according to claim 1 wherein said absorbent structure comprises a tissue laminate joined by soldering a first tissue layer to a second tissue layer, preferably said first tissue layer and said second tissue layer both being provided by folding a tissue upon itself.

5. Process according to any of the preceding claims wherein said disposable absorbent articles are sanitary napkins or panty liners used in the crotch portion of an undergarment, preferably said articles have side flaps extending beyond and wrapping around the side edge of the crotch portion of said undergarment.

## Patentansprüche

1. Verfahren zur Herstellung wegwerfbarer absorbierender Artikel, wobei die Artikel eine absorbierende Struktur umfassen, wobei die absorbierende Struktur eine erste und eine zweite äußerste Oberfläche umfasst, welche sich an gegenüberliegenden Seiten der absorbierenden Struktur befinden, wobei das Verfahren die folgenden Schritte umfasst:
- Bereitstellen eines ersten und eines zweiten Materials sowie eines Lötmittels, wobei das Lötmittel bei 40°C nicht klebrig ist;
- Befördern des ersten und zweiten Materials in einer Maschinenrichtung mit einer Oberflächengeschwindigkeit von mindestens 0,4 m/s;
- Verbinden des ersten und zweiten Materials durch
- Erhitzen des Lötmittels auf eine Temperatur oberhalb der Verfestigungstemperatur des Lötmittels,
- Aufbringen des Lötmittels auf ein oder beide Material/ien bevor das Lötmittel unter seine Verfestigungstemperatur abkühlt,
- In-Kontakt-Bringen des ersten und zweiten Materials bevor das Lötmittel unter seine Verfestigungstemperatur abkühlt, um eine permanente Verbindung zu bilden, und
- wobei das erste Material ein flüssigkeitsdurchlässiges Deckblatt ist und das zweite Material ein Rückenblatt, vorzugsweise ein flüssigkeitsundurchlässiges Rückenblatt, ist, oder
- wobei das erste Material ein flüssigkeitsdurchlässiges Deckblatt ist und das zweite Material die absorbierende Struktur ist und das Deckblatt und die absorbierende Struktur über die erste äußerste Oberfläche hin verbunden sind, oder
- wobei das erste Material eine erste Schicht eines mehrschichtigen, flüssigkeitsdurchlässigen Deckblattes ist und das zweite Material eine zweite Schicht eines mehrschichtigen, flüssigkeitsdurchlässigen Deckblatts ist, oder
- wobei das erste Material ein atmungsaktives Rückenblatt ist und das zweite Material die absorbierende Struktur ist und das Rückenblatt und die absorbierende Struktur über die zweite äußerste Oberfläche hin verbunden sind, oder
- wobei das erste Material eine erste Schicht eines mehrschichtigen Rückenblatts ist und das zweite Material eine zweite Schicht eines mehrschichtigen Rückenblatts ist, wobei vorzugsweise das Rückenblatt flüssigkeitsundurchlässig ist, oder
- wobei die absorbierende Struktur mehrere Schichten umfasst und das erste Material eine erste Schicht der absorbierenden Struktur ist und das zweite Material eine zweite Schicht der absorbierenden Struktur ist.

2. Verfahren nach Anspruch 1, wobei die Materialien eine Oberflächengeschwindigkeit von mindestens 1,5 m/s in Maschinenrichtung haben.

3. Verfahren nach Anspruch 1, wobei das Deckblatt und das Rückenblatt einen Umfangsrandabschnitt umfassen, der sich über den Umfang der absorbierenden Struktur hinaus erstreckt, und das Löten das Rückenblatt mit dem Deckblatt in mindestens einem Teil des Umfangsrandabschnitts, vorzugsweise entlang dem gesamten Umfangsrandabschnitt, verbindet.

4. Verfahren nach Anspruch 1, wobei die absorbierende Struktur ein Tissue-Laminat umfasst, das durch Löten einer ersten Tissue-Schicht an eine zweite Tissue-Schicht verbunden wurde, wobei vorzugsweise die erste Tissue-Schicht und die zweite Tissue-Schicht beide durch Falten eines Tissues auf sich selbst bereitgestellt sind.

5. Verfahren nach einem der vorherigen Ansprüche, wobei die wegwerfbaren absorbierenden Artikel Hygienevorlagen oder Slipeinlagen sind, die im Schrittbereich einer Unterbekleidung verwendet werden, wobei vorzugsweise die Artikel Seitenlappen aufweisen, die sich über den Seitenrand des Schrittbereichs der Unterbekleidung erstrecken und um diesen umgeschlagen werden.

## Revendications

1. Procédé de fabrication d'articles absorbants jetables, lesdits articles comprenant une structure absorbante, ladite structure absorbante comprenant une première surface et une deuxième surface les plus à l'extérieur, situées sur des côtés opposés de ladite structure absorbante, ledit procédé comprenant les étapes qui consistent à :
- se procurer un premier matériau et un deuxième matériau, et un agent de soudage, ledit agent de soudage étant non collant à 40°C,
- transporter lesdits premier et deuxième matériaux dans un sens machine, à une vitesse linéaire d'au moins 0,4m/s ;
- assembler ledit premier matériau et ledit deuxième matériau en
o chauffant l'agent de soudage à une température supérieure à la température de solidification dudit agent de soudage
o appliquant ledit agent de soudage à l'un ou aux deux matériaux avant que ledit agent de soudage ne refroidisse en dessous de sa température de solidification,
o amenant ledit premier et ledit second matériau en contact l'un avec l'autre pour réaliser une liaison permanente, et
- ledit premier matériau est une feuille de dessus perméable aux liquides et ledit deuxième matériau est une feuille de fond, de préférence une feuille de fond imperméable aux liquides,
- ledit premier matériau est une feuille de dessus perméable aux liquides et ledit deuxième matériau est ladite structure absorbante, et ladite feuille de dessus et ladite structure absorbante sont assemblées au niveau de ladite première surface la plus à l'extérieur,
- ledit premier matériau est une première couche d'une feuille de dessus multicouche, perméable aux liquides, et ledit deuxième matériau est une deuxième couche d'une feuille de dessus multicouche, perméable aux liquides,
- ledit premier matériau est une feuille de fond apte à respirer et ledit deuxième matériau est ladite structure absorbante, et ladite feuille de fond et ladite structure absorbante sont assemblées au niveau de ladite deuxième surface la plus à l'extérieur,
- ledit premier matériau est une première couche d'une feuille de fond multicouche et ledit deuxième matériau est une deuxième couche d'une feuille de fond multicouche, ladite feuille de fond étant, de préférence, imperméable aux liquides,
- ladite structure absorbante comprend plusieurs couches, et ledit premier matériau est une première couche de ladite structure absorbante et ledit deuxième matériau est une deuxième couche de ladite structure absorbante.

2. Procédé selon la revendication 1, dans lequel lesdits matériaux ont une vitesse linéaire d'au moins 1,5 m/s, dans le sens machine.

3. Procédé selon la revendication 1, dans lequel ladite feuille de dessus et ladite feuille de fond comprennent une partie de bord périphérique s'étendant au-delà de la périphérie de ladite structure absorbante, et ladite opération de soudage assemble ladite feuille de fond à ladite feuille de dessus sur au moins une portion de ladite partie de bord périphérique, de préférence le long de toute la partie de bord périphérique.

4. Procédé selon la revendication 1, dans lequel ladite structure absorbante est constituée d'un stratifié de papier tissu assemblé par soudage d'une première couche de papier tissu à une deuxième couche de papier tissu, ladite première couche de papier tissu et ladite deuxième couche de papier tissu étant, de préférence, toutes les deux fournies par repliage d'un papier tissu sur lui-même.

5. Procédé selon l'une quelconque des revendications précédentes, dans lequel lesdits articles absorbants jetables sont des serviettes hygiéniques ou des garnitures de culotte utilisées dans la partie d'entrejambe d'un sous-vêtement, lesdits articles ayant, de préférence, des rabats latéraux s'étendant au-delà du bord latéral de la partie d'entrejambe dudit sous-vêtement et enveloppant ce bord latéral.
